# FASCICULE DE BREVET EUROPEEN

(11) **EP 2 685 894 B1**
(45) Date de publication et mention de la délivrance du brevet: **08.04.2015**
(21) Numéro de dépôt: 12704841.1
(22) Date de dépôt: 22.02.2012
(51) Int. Cl.: A61B 5/117, G06K 9/20, G06K 9/00

(54) **CAPTEUR DE RÉSEAUX VEINEUX D'UNE PARTIE D'UN CORPS VIVANT**
SENSOR FÜR VENENNETZE EINES TEILS EINES LEBENDEN KÖRPERS
SENSOR FOR VENOUS NETWORKS OF A PART OF A LIVING BODY

(30) Priorité: 18.03.2011 FR 1152263
(43) Date de publication de la demande: 22.01.2014
(73) Titulaire: Morpho, 92130 Issy-les-Moulineaux (FR)
(72) Inventeur: MONTEILLIET, Gilles, F-75015 Paris (FR); DARBOIS, Matthieu, F-75015 Paris (FR); CRUCHAGA, Michel, F-75015 Paris (FR); PICARD, Sylvaine, F-75015 Paris (FR)
(74) Mandataire: Maillet, Alain
(86) Numéro de dépôt international: PCT/EP2012/053021
(87) Numéro de publication internationale: WO 2012/126691

(56) Documents cités:
- EP-A2- 2 241 998
- JP-A- 2008 054 787
- US-A1- 2005 047 632
- US-A1- 2008 063 243
- US-A1- 2010 092 047

## Description

La présente invention concerne un capteur de réseaux veineux d'une partie d'un corps vivant, ainsi qu'un procédé d'asservissement d'un tel capteur.

Un capteur de réseaux veineux d'une partie d'un corps vivant tel que celui d'un doigt, d'une main, d'un orteil, etc. est très utilisé, par exemple pour le contrôle d'accès à un site ou à une machine.

Un capteur de réseaux veineux comporte une source d'éclairement infrarouge qui éclaire un moyen d'acquisition d'images du capteur tel que par exemple une caméra CCD.

Cette source d'éclairement infrarouge est réalisée par un ensemble d'émetteurs infrarouges qui sont habituellement positionnés les uns par rapport aux autres selon des lignes et/ou des colonnes. On parle alors de barrettes ou de matrices d'émetteurs infrarouges.

Lorsqu'un corps vivant est placé entre la source d'éclairement infrarouge et le moyen d'acquisition d'images, certains rayons du flux lumineux frappent directement le moyen d'acquisition d'images et d'autres frappent ce moyen d'acquisition d'images après avoir traversé le corps vivant. L'image ainsi acquise est alors traitée numériquement pour que le réseau veineux du corps vivant apparaisse sur cette image. L'authentification d'un utilisateur peut alors être réalisée par comparaison du réseau veineux ainsi acquis et du réseau veineux qu'il a préalablement enregistré.

Si le principe d'un capteur de réseaux veineux est simple, sa mise en oeuvre peut poser le problème de surexposition de l'image acquise. En effet, les rayons qui frappent directement le moyen d'acquisition d'images induisent une intensité des pixels de l'image qui est importante comparée à celle induite par les rayons qui traversent le corps vivant. Cette différence d'intensité provoque des artéfacts sur l'image acquise, tels qu'un halo lumineux ou des surintensités locales, surtout aux bords du corps vivant. Ceci est particulièrement vrai pour les systèmes de capture à la volée car la position de l'objet à acquérir peut être contrainte. Ces artéfacts perturbent les traitements qui sont appliqués à l'image acquise pour faire apparaître le réseau veineux.

Pour améliorer la qualité de l'acquisition du réseau veineux, différentes solutions ont été proposées. Par exemple, on connaît un capteur de réseaux veineux dans lequel l'activation des émetteurs infrarouges est liée à la détection de la présence d'un corps vivant.

Un tel capteur de réseaux veineux ne donne pas entière satisfaction.

Les documents US-A-2005/047632, US-A-2008/06243 et US-A-2010/092047 divulguent des capteurs de réseaux veineux.

Un objet de la présente invention est de proposer un capteur de réseaux veineux d'une partie d'un corps vivant qui ne présente pas les inconvénients de l'art antérieur et qui en particulier évite de saturer le moyen d'acquisition d'images que ce soit en présence ou en absence du corps vivant.

A cet effet, est proposé un capteur de réseaux veineux d'une partie d'un corps vivant comportant:
- une source d'éclairement infrarouge, et
- un moyen d'acquisition d'images,
ledit capteur étant caractérisé en ce qu'il comporte en outre un guide d'onde éclairé par ladite source d'éclairement infrarouge et présentant au moins une zone d'extraction destinée à extraire les rayons infrarouges dudit guide d'onde par une face d'extraction du guide d'onde selon au moins une direction principale d'extraction,
en ce que ledit moyen d'acquisition d'images est disposé en vis-à-vis de ladite face d'extraction de manière à définir entre eux un passage par l'entrée duquel ladite partie du corps vivant peut pénétrer, et comportant un élément sensible, et
en ce que la ou chaque zone d'extraction est telle que la ou chaque direction principale d'extraction est orientée de manière à ne pas intercepter ledit élément sensible.

Selon un mode de réalisation particulier, ladite ou chaque zone d'extraction est portée par la face opposée à la face d'extraction.

Selon un autre mode de réalisation particulier, ladite ou chaque zone d'extraction est portée par la face d'extraction.

Avantageusement, ladite ou au moins une desdites zones d'extraction est constituée d'un pan incliné réalisé dans ladite face d'extraction.

Avantageusement, ladite ou au moins une desdites zones d'extraction est constituée de microprismes.

Avantageusement, le pan incliné est en amont des microprismes dans le sens de progression des rayons infrarouges dans le guide d'onde.

Selon un mode de réalisation particulier, la source lumineuse est disposée à l'opposé de l'entrée du passage, le capteur comporte un moyen de collimation disposé entre la source d'éclairement et le guide d'onde et prévu pour collimater au voisinage de l'infini, les rayons infrarouges issus de la source d'éclairement, et le guide d'onde présente sur le trajet des rayons infrarouges ainsi collimatés, une surface réfléchissante présentant un angle d'inclinaison prévu pour replier les rayons infrarouges transmis et les dévier vers la ou chaque zone d'extraction.

Selon un autre mode de réalisation particulier, la source lumineuse est disposée à l'opposé de l'entrée du passage, le capteur comporte un moyen de focalisation disposé entre la source d'éclairement et le guide d'onde et prévu pour focaliser les rayons infrarouges issus de la source d'éclairement vers un point de focalisation, le guide d'onde présente sur le trajet des rayons infrarouges ainsi focalisés, une surface réfléchissante disposée à distance du point de focalisation et prévue pour réfléchir les rayons infrarouges et provoquer leur déviation vers la zone d'extraction.

Selon un autre mode de réalisation particulier, ledit guide d'onde présente une première zone d'extraction et une deuxième zone d'extraction, ladite source d'éclairement infrarouge présente un premier élément éclairant éclairant la tranche du guide d'onde disposée du côté de l'entrée du passage, et un deuxième élément éclairant éclairant la tranche du guide d'onde disposée du côté opposé à l'entrée du passage, la première zone d'extraction est disposée entre le premier élément éclairant et la deuxième zone d'extraction et est prévue pour extraire les rayons infrarouges issus du premier élément éclairant, la deuxième zone d'extraction est disposée entre le deuxième élément éclairant et la première zone d'extraction, et est prévue pour extraire les rayons infrarouges issus du deuxième élément éclairant, et les zones d'extraction sont telles que les rayons infrarouges extraits par la première zone d'extraction et les rayons infrarouges extraits par la deuxième zone d'extraction convergent les uns vers les autres au niveau de l'endroit du passage où est susceptible de se positionner la partie.

Avantageusement, le capteur comporte une plaque de protection placée en vis-à-vis de la face opposée à la face d'extraction et constituée d'un matériau empêchant le passage des rayons infrarouges mais laissant passer les rayons du spectre visible.

Avantageusement, le capteur comporte une plaque de filtrage placée entre la zone d'extraction et le passage et constituée d'un matériau empêchant le passage des rayons du spectre visible mais laissant passer les rayons infrarouges.

Avantageusement, le capteur comporte des moyens d'asservissement prévus pour commander en puissance la source d'éclairement.

Avantageusement, les moyens d'asservissement sont constitués par une unité de commande prévue pour commander la source d'éclairement et au moins un moyen de détection prévu pour envoyer à ladite unité de commande, une valeur représentative de la puissance d'éclairement qu'il reçoit, l'unité de commande comportant des moyens pour commander la source d'éclairement en fonction de ladite valeur.

Selon un mode de réalisation particulier, le ou chaque moyen de détection est placé au voisinage de l'élément sensible.

Selon un autre mode de réalisation particulier, le ou chaque moyen de détection est disposé au voisinage de la ou des zones d'extraction.

Avantageusement, le ou chaque moyen de détection est disposé au fond d'un trou réalisé dans le guide d'onde, chacun desdits trous présentant un axe sensiblement parallèle à la direction d'extraction.

Avantageusement, la surface du ou de chaque trou est recouverte d'un matériau imperméable aux rayons infrarouges.

L'invention propose également un procédé d'asservissement d'un capteur selon certaines des variantes précédentes, ledit procédé comprenant:
- une étape d'initialisation, au cours de laquelle le moyen de détection envoie à l'unité de commande, la valeur de référence représentative de la puissance lumineuse qu'il reçoit en l'absence de la partie,
- une étape d'envoi au cours de laquelle le moyen de détection envoie à l'unité de commande, une valeur représentative de la puissance lumineuse qu'il reçoit,
- une étape de calcul au cours de laquelle l'unité de commande calcule l'écart entre la valeur ainsi reçue et la valeur de référence,
- une étape de test, au cours de laquelle l'unité de commande vérifie si l'écart ainsi calculé est supérieur, en valeur absolue, à un écart prédéterminé,
- si l'écart ainsi calculé reste inférieur à l'écart prédéterminé, une étape de bouclage au cours de laquelle le processus boucle sur l'étape d'envoi,
- si l'écart ainsi calculé est supérieur à l'écart prédéterminé, une étape de commande au cours de laquelle l'unité de commande commande une augmentation de la puissance lumineuse émise par la source d'éclairement, et
- une étape de retour au cours de laquelle le processus boucle sur l'étape d'envoi.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels :
la Fig. 1 représente une vue de côté d'un capteur de réseaux veineux selon un premier mode de réalisation de l'invention,
la Fig. 2 représente une vue de côté d'un capteur de réseaux veineux selon un deuxième mode de réalisation de l'invention,
la Fig. 3 représente une vue de côté d'un capteur de réseaux veineux selon un troisième mode de réalisation de l'invention,
la Fig. 4 représente une vue de côté d'un capteur de réseaux veineux selon un quatrième mode de réalisation de l'invention,
la Fig. 5 représente une vue de côté d'un capteur de réseaux veineux selon un cinquième mode de réalisation de l'invention,
la Fig. 6 représente une vue de côté d'un capteur de réseaux veineux selon un sixième mode de réalisation de l'invention,
la Fig. 7 représente une vue de côté d'un capteur de réseaux veineux selon un septième mode de réalisation de l'invention en l'absence du corps vivant,
la Fig. 8 représente une vue de côté d'un capteur de réseaux veineux de la Fig. 7 en présence du corps vivant,
la Fig. 9 représente une vue de côté d'un capteur de réseaux veineux selon un huitième mode de réalisation de l'invention,
la Fig. 10 représente une vue de dessous du capteur de réseaux veineux de la Fig. 9,
la Fig. 11 représente un algorithme d'asservissement du capteur de la Fig. 7 ou de la Fig. 9,
la Fig. 12 représente une vue de côté d'un capteur de réseaux veineux selon un neuvième mode de réalisation de l'invention, et
la Fig. 13 représente une vue de côté d'un capteur de réseaux veineux selon un dixième mode de réalisation de l'invention.

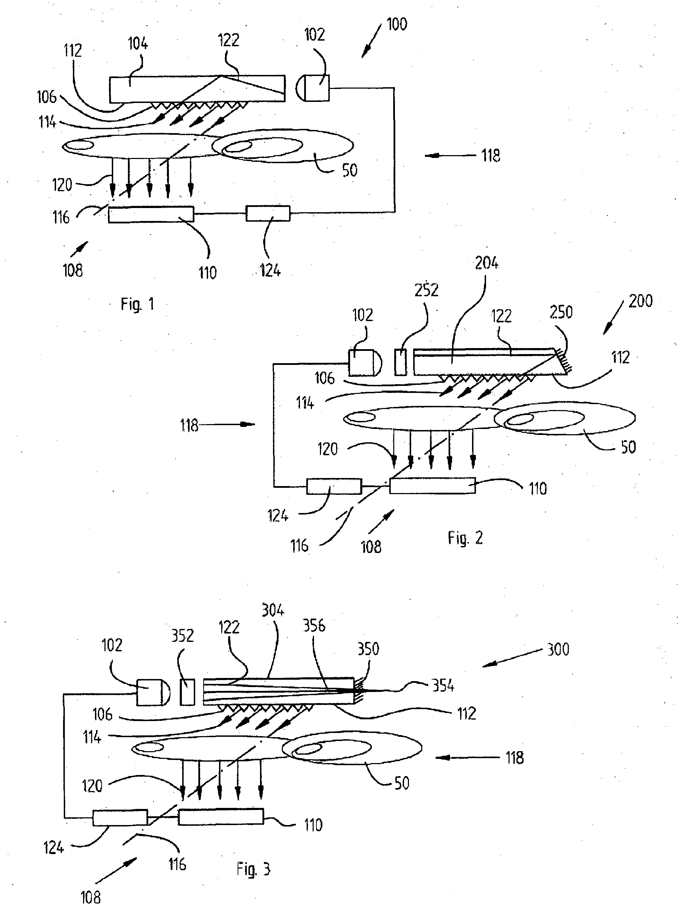

La Fig. 1 montre un capteur 100 prévu pour capturer une image des réseaux veineux d'une partie 50 à la volée ou de manière statique, ici un doigt, d'un corps vivant.

Le capteur 100 comporte:
- une source d'éclairement infrarouge 102,
- un guide d'onde 104 éclairé par ladite source d'éclairement infrarouge 102 et présentant sur une face 112, au moins une zone d'extraction 106 destinée à extraire les rayons infrarouges 114 dudit guide d'onde 104 selon au moins une direction principale d'extraction 116,
- un moyen d'acquisition d'images 108 disposé en vis-à-vis de ladite face 112 de manière à définir entre eux un passage 118 par l'entrée duquel ladite partie 50 du corps vivant peut pénétrer, et comportant un élément sensible 110,
la ou chaque zone d'extraction 106 étant telle que la ou chaque direction principale d'extraction 116 de ladite zone d'extraction 106 est orientée de manière à ne pas intercepter ledit élément sensible 110.

La face 112 où est disposée la zone d'extraction 106 constitue la face d'extraction 112 par où sont extraits les rayons infrarouges 114.

La source d'éclairement 102 est par exemple une barrette de diodes.

Le guide d'onde 104 est par exemple une lame de PMMA (Polyméthacrylate de méthyle) dont les quatre tranches ont été polies et dont au moins l'une des tranches est éclairée par la source d'éclairement 102.

Dans le mode de réalisation de l'invention présenté sur la Fig. 1, une seule tranche en épaisseur est éclairée et la face 112 qui porte la zone d'extraction 106 est perpendiculaire à cette tranche.

Afin d'augmenter les réflexions internes au guide d'onde 104 et surtout de minimiser les fuites lumineuses vers l'extérieur, les trois autres tranches sont recouvertes de couches successives d'adhésif en PVC non tramé (par exemple, l'adhésif de la société TESA portant la référence: tesa® Ruban PVC 4163) et d'adhésif aluminium (par exemple, l'adhésif de la société TESA portant la référence: tesa® Ruban aluminium 50565).

Le moyen d'acquisition d'images 108 est par exemple du type caméra CCD et il comporte un élément sensible qui peut être lui-même divisé en plusieurs sous-éléments sensibles.

Un élément est sensible au sens où il délivre une information représentative de la puissance lumineuse qu'il reçoit. L'information ainsi délivrée est ensuite transmise à une unité de commande 124 qui reconstruit une image du réseau veineux ainsi observé. L'image ainsi reconstruite pourra ensuite être utilisée dans le cadre d'une procédure d'authentification par comparaison de cette image avec des images de références.

L'unité de commande 124 commande également l'allumage de la source d'éclairement 102. Le déclenchement de la commande de l'allumage peut être soumis à la détection de la présence de la partie du corps 50, par exemple par la mise en place d'une barrière infrarouge. Dans un autre cas particulier, il est possible que la source d'éclairement 102 soit allumée en permanence.

La zone d'extraction 106 se présente ici sous la forme d'un patch qui est optiquement couplé à la face 112 du guide d'onde 104. C'est-à-dire que la zone d'extraction 106 est sur la face d'extraction 112.

Le patch est destiné à orienter les rayons infrarouges 114 extraits de la manière appropriée. Le patch comporte ici des prismes, et il peut être par exemple un film polyester ou polycarbonate supportant une structure superficielle rainurée en forme de microprismes à profil symétrique ou asymétrique, comme par exemple le film de la société Luminit portant la référence DTF. La hauteur des microprismes symétriques est par exemple de l'ordre de 100 µm. L'angle des microprismes est choisi pour obtenir l'angle voulu pour les rayons infrarouges 114 extraits.

Dans le mode de réalisation présenté ici, les microprismes sont accolés sur le guide d'onde 104 par leurs bases, par l'intermédiaire d'un moyen de couplage optique comme par exemple un liquide d'indice ou un adhésif optique transparent qui améliore le couplage entre les deux et permet l'extraction des rayons infrarouges 114 vers l'extérieur du guide d'onde 104.

Bien sûr, il est possible de prévoir d'autres moyens pour permettre l'extraction des rayons infrarouges 114. En particulier, il est possible de graver des microprismes dans le guide d'onde 104.

Pour chaque zone élémentaire d'extraction, c'est-à-dire ici de chaque microprisme, le profil de la luminance des rayons infrarouges issus de cette zone élémentaire d'extraction se présente sous la forme d'un lobe dont la direction principale d'extraction 116 constitue la direction de la luminance maximale.

Le flux nominal est de l'ordre de 1W, comme par exemple dans le cas d'un capteur d'images 110 du type DALSA 4M60, avec un passage 118 d'une largeur de l'ordre de 6 cm et une source lumineuse 102 constituée de diodes comme par exemple les diodes de la société OSRAM portant la référence SFH4235-EB.

La totalité du flux lumineux extrait du guide d'onde 104 n'est pas envoyée vers l'élément sensible 110.

Les rayons infrarouges issus de la source d'éclairement 102 se propagent dans le guide d'onde 104 sous forme de rayons transmis 122 et après extraction du guide d'onde 104, sous forme de rayons infrarouges extraits 114.

En l'absence du doigt 50, les rayons infrarouges 114 extraits par chaque microprisme sont émis selon un lobe d'émission principal qui n'intercepte pas directement l'élément sensible 110 ce qui évite sa saturation. En présence du doigt 50, les rayons infrarouges extraits 114 sont diffusés à l'intérieur du doigt 50 et absorbés par l'hémoglobine avant de ressortir sous forme de rayons sortants 120 qui sont interceptés par l'élément sensible 110 mais qui ont une puissance lumineuse réduite qui ne provoque pas la saturation de celui-ci.

Dans la suite de la description, différents modes de réalisation de l'invention sont décrits. Pour chacun de ces modes de réalisation, les éléments communs avec le mode de réalisation de l'invention de la Fig. 1 portent les mêmes références.

La Fig. 2 montre un deuxième mode de réalisation de l'invention.

Dans le mode de réalisation de l'invention de la Fig. 1, la source d'éclairement 102 est disposée du côté de l'entrée du passage 118. Une telle implantation peut induire un échauffement du capteur 100 au niveau de cette entrée, au risque de brûler ladite partie 50.

La Fig. 2 montre un capteur 200 ayant une implantation dans laquelle la source lumineuse 102 est à l'opposé de l'entrée du passage 118.

Dans la mesure où, pour obtenir les meilleurs résultats, l'éclairement doit venir de l'arrière de la main, il est nécessaire que les rayons lumineux 122 à l'intérieur du guide d'onde 204 subissent un repliement.

Le capteur 200 comporte ainsi un moyen de collimation 252 disposé entre la source d'éclairement 102 et le guide d'onde 204 et qui est prévu pour collimater au voisinage de l'infini, les rayons infrarouges issus de la source d'éclairement 102 et transmis à l'intérieur du guide d'onde 204.

Le guide d'onde 204 présente sur le trajet des rayons infrarouges 122 ainsi collimatés à l'intérieur du guide d'onde 204, une surface réfléchissante 250 présentant un angle d'inclinaison prévu pour retourner les rayons infrarouges transmis 122 dans le guide d'onde 204 et les dévier vers la zone d'extraction 106.

Dans le mode de réalisation de l'invention présenté sur la Fig. 2, la surface réfléchissante 250 est réalisée sur la tranche opposée à la tranche éclairée par la source lumineuse 102.

La Fig. 3 montre un capteur 300 ayant également une implantation dans laquelle la source lumineuse 102 est à l'opposé de l'entrée du passage 118.

Le capteur 300 comporte un moyen de focalisation 352 disposé entre la source d'éclairement 102 et le guide d'onde 304 et qui est prévu pour focaliser les rayons infrarouges issus de la source d'éclairement 102 et transmis à l'intérieur du guide d'onde 304 vers un point de focalisation 354.

Le guide d'onde 304 présente sur le trajet des rayons infrarouges 122 ainsi focalisés à l'intérieur du guide d'onde 304, une surface réfléchissante 350 qui est disposée à distance du point de focalisation 354.

Lorsque les rayons infrarouges 122 rencontrent la surface réfléchissante 350, ils sont réfléchis vers un point de réflexion 356 qui provoque leur retournement dans le guide d'onde 304 et les dévie vers la zone d'extraction 106.

Dans le mode de réalisation de l'invention présenté sur la Fig. 3, la surface réfléchissante 350 est réalisée sur la tranche opposée à la tranche éclairée par la source lumineuse 102, et le point de focalisation 354 est situé au-delà de la surface réfléchissante 350 dans le sens de propagation des rayons infrarouges depuis la source d'éclairement 102.

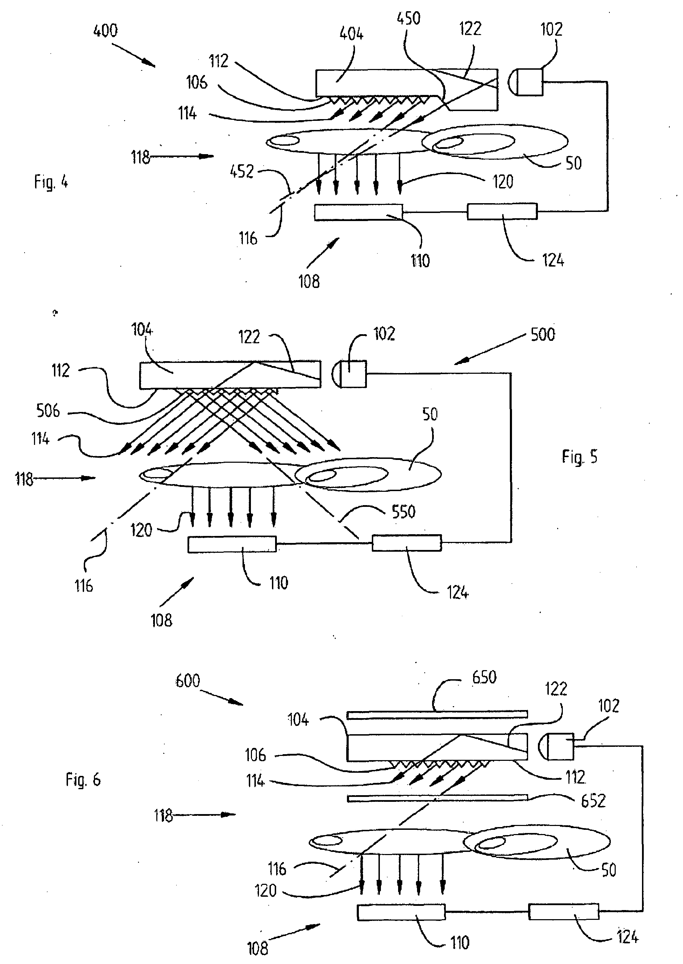

La Fig. 4 montre un capteur 400 dont la zone d'extraction 450 est constituée par un plan incliné qui est ici un pan incliné réalisé dans la face 112. Les microprismes des modes de réalisation précédents sont donc ici remplacés par le pan incliné 450 qui permet d'extraire les rayons infrarouges 122 du guide d'onde 404 selon une direction principale d'extraction 452 qui n'intercepte pas l'élément sensible 110.

Dans le mode de réalisation de la Fig. 4, la source d'éclairement 102 est du côté de l'entrée du passage 118, mais il est possible de combiner les modes de réalisation des Figs. 2 et 3 afin de déporter la source d'éclairement 102 du côté opposé.

La direction principale d'extraction 452 est confondue avec la normale au pan incliné 450.

Avec les microprismes, une fraction des rayons infrarouges 114 ne fuit pas selon la direction principale d'extraction 116. Les surfaces des microprismes sont alors lumineuses du point de vue de l'élément sensible 110. Même si cela n'est pas gênant, certaines applications peuvent nécessiter la réduction de ces perturbations.

La mise en place du pan incliné 450 qui constitue une surface lenticulaire permettant de contrôler le lobe de luminance des rayons infrarouges permet de réduire ces phénomènes.

Il est également possible de combiner le principe d'une zone d'extraction 106 constituée de microprismes et une zone d'extraction 450 constituée du pan incliné 450 afin de permettre un contrôle fin de l'éclairage du doigt 50.

Dans ce dernier mode de réalisation, le pan incliné 450 est en amont des microprismes dans le sens de progression des rayons infrarouges 122 dans le guide d'onde 404 pour permettre de renforcer l'éclairage à l'arrière du doigt 50 qui est la zone la plus pertinente du point de vue de la reconnaissance par réseau veineux.

La Fig. 5 montre un capteur 500 similaire à celui de la Fig. 1, mais dans lequel la zone d'extraction 506 est telle qu'elle est prévue pour extraire les rayons infrarouges 114 du guide d'onde 104 selon une pluralité de directions principales d'extraction 116 et 550.

Ce mode de réalisation permet d'éclairer un grand volume.

Dans le mode de réalisation de la Fig. 5, il y a deux directions principales d'extraction 116 et 550 qui sont sensiblement orthogonales l'une à l'autre, mais qui comme précédemment n'interceptent pas l'élément sensible 110.

La zone d'extraction 506 est ici réalisée à partir de microprismes à double lobes d'émission.

La zone d'extraction 506 est par exemple constituée d'un film de la société Vikuiti 3 M portant la référence BEF.

Dans un autre mode de réalisation, chaque microprisme possède deux pentes d'extraction à 20° et la direction principale d'extraction est alors à 70° par rapport à la face d'extraction 112 et les deux lobes d'émission présentent alors un angle de 40° l'un par rapport à l'autre.

Bien sûr, il est possible de combiner le mode de réalisation de la Fig. 5 avec les modes de réalisation des Figs. 1 à 4.

La Fig. 6 montre un capteur 600 qui est similaire à celui de la Fig. 1.

Pour faciliter la mise en place de sa partie du corps 50, l'utilisateur doit pouvoir voir sa partie de corps 50 à travers le guide d'onde 104. A cette fin, il peut se placer au dessus du guide d'onde 104 et voir à travers la face supérieure qui est la face opposée à la face 112 portant la zone d'extraction 106.

Pour limiter la transmission de rayons infrarouges vers l'extérieur du guide d'onde 104 par la face supérieure, une plaque de protection 650 est disposée en vis-à-vis de ladite face supérieure. Cette plaque de protection 650 est constituée d'un matériau empêchant le passage des rayons infrarouges mais laissant passer au moins partiellement les rayons du spectre visible.

Cette plaque de protection 650 peut être par exemple réalisée dans un verre architectural de la société Schott DESAG portant la référence IMERA D4218.

Dans le cas d'une procédure d'authentification, il est possible de combiner l'analyse du réseau veineux et l'analyse de l'empreinte digitale du doigt 50.

Un dispositif complémentaire est alors nécessaire. Ce dispositif complémentaire comprend une source de lumière visible qui éclaire la face inférieure du doigt 50 et un moyen d'acquisition adapté qui capture l'image de l'empreinte de ce doigt 50 ainsi éclairé.

Mais dans le cadre d'une capture de l'image du réseau veineux, il est préférable d'éviter que la zone d'extraction 106 soit illuminée par les rayons lumineux émis par la source de lumière visible.

A cette fin, une plaque de filtrage 652 est disposée entre la zone d'extraction 106 et le passage 118, c'est-à-dire entre la zone d'extraction 106 et le doigt 50. Cette plaque de filtrage 652 est constituée d'un matériau empêchant le passage des rayons du spectre visible mais laissant passer les rayons infrarouges. Le doigt 50 passe donc entre la plaque de filtrage 652 et l'élément sensible 110.

Cette plaque de filtrage 652 peut être par exemple réalisée dans un verre architectural de la société Schott DESAG portant la référence IMERA D4403.

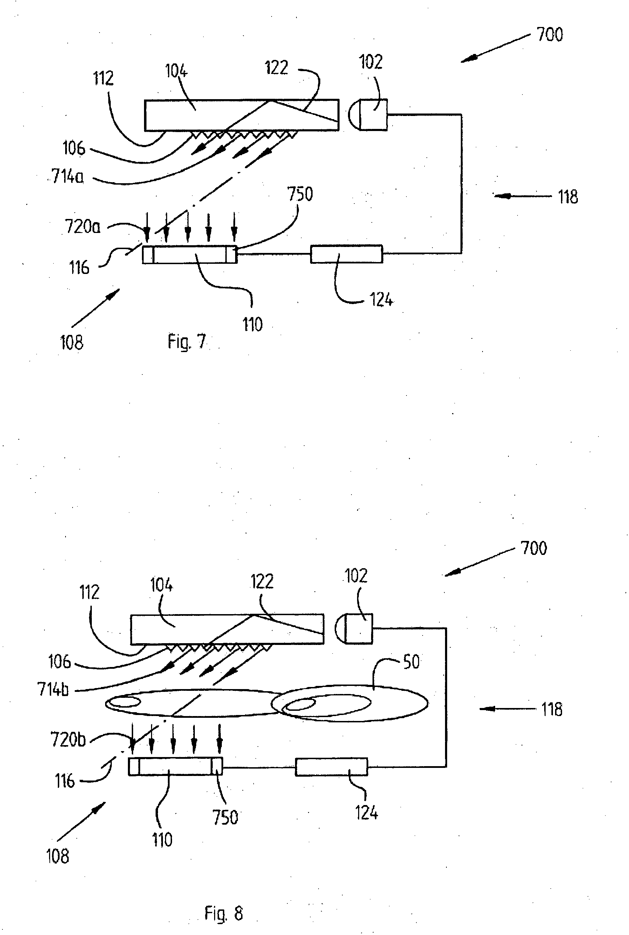

La Fig. 7 et la Fig. 8 montrent un capteur 700 comportant des moyens d'asservissement de la source d'éclairage 102 pour commander en puissance la source d'éclairement 102 selon que le doigt 50 soit ou non présent dans le passage 118. Comme pour les autres modes de réalisation, ce nouveau mode de réalisation est présenté dans le cadre d'un capteur 700 similaire à celui de la Fig. 1, mais il peut s'appliquer aux modes de réalisation des autres Figs.

Sur la Fig. 7, le doigt 50 n'est pas présent et sur la Fig. 8, le doigt 50 est présent.

Le capteur 700 comporte au voisinage de l'élément sensible 110, au moins un moyen de détection 750 qui est par exemple du type photo-détecteur et qui n'est pas intercepté par les rayons infrarouges 714a, 714b extraits du guide d'onde 104 selon la direction principale d'extraction 116.

Les moyens d'asservissement sont constitués ici par le moyen de détection 750 et l'unité de commande 124.

En l'absence du doigt 50 et du fait de la diffusion des rayons infrarouges 714a émis par la source d'éclairement 102 et transmis par la zone d'extraction 106, le moyen de détection 750 reçoit une portion 720a de ces rayons infrarouges 714a.

Au cours d'une étape d'initialisation, le moyen de détection 750 envoie à l'unité de commande 124, une valeur de référence représentative de la puissance lumineuse qu'il reçoit en l'absence du doigt 50.

Au cours d'un fonctionnement normal, le moyen de détection 750 envoie régulièrement à l'unité de commande 124, une valeur représentative de la puissance lumineuse qu'il reçoit.

Si la valeur nouvellement reçue ne s'écarte pas de la valeur de référence de plus d'un écart prédéterminé, c'est-à-dire si le doigt 50 n'est pas présent, rien ne se passe.

Si la valeur nouvellement reçue s'écarte de la valeur de référence de plus que l'écart prédéterminé, c'est-à-dire si le doigt 50 est présent et qu'une baisse de la puissance lumineuse reçue par le moyen de détection 750 est observée, l'unité de commande 124 commande la source d'éclairement 102 de manière à augmenter la puissance lumineuse émise tant que la valeur envoyée par le moyen de détection 750 reste éloignée de la valeur de référence.

Selon un autre mode de réalisation de l'invention, il est possible que la géométrie du capteur soit telle que le lobe d'émission est étroit et que le moyen de détection 750 ne reçoit aucun flux lumineux en l'absence du doigt 50. La mise en position du doigt 50 va alors entraîner une augmentation de la puissance lumineuse reçue par le moyen de détection 750 et en réaction, l'unité de commande 124 commandera la source d'éclairement 102 de manière à diminuer la puissance lumineuse émise.

La Fig. 8 montre le cas où le doigt 50 est présent et où la puissance émise par la source d'éclairement 102 a été augmentée. Les rayons infrarouges 714b transmis à travers la zone d'extraction 106 ont alors une puissance supérieure tandis que la puissance des rayons infrarouges 720b qui ont traversé le doigt 50 a sensiblement la même puissance lumineuse que celle des rayons infrarouges 720a correspondants au cas où le doigt 50 est absent.

L'unité de commande 124 comporte des moyens pour commander la source d'éclairement 102 en fonction de la valeur qu'il reçoit du moyen de détection 750.

Ainsi, l'asservissement tient compte de la perméabilité du doigt 50 aux rayons infrarouges.

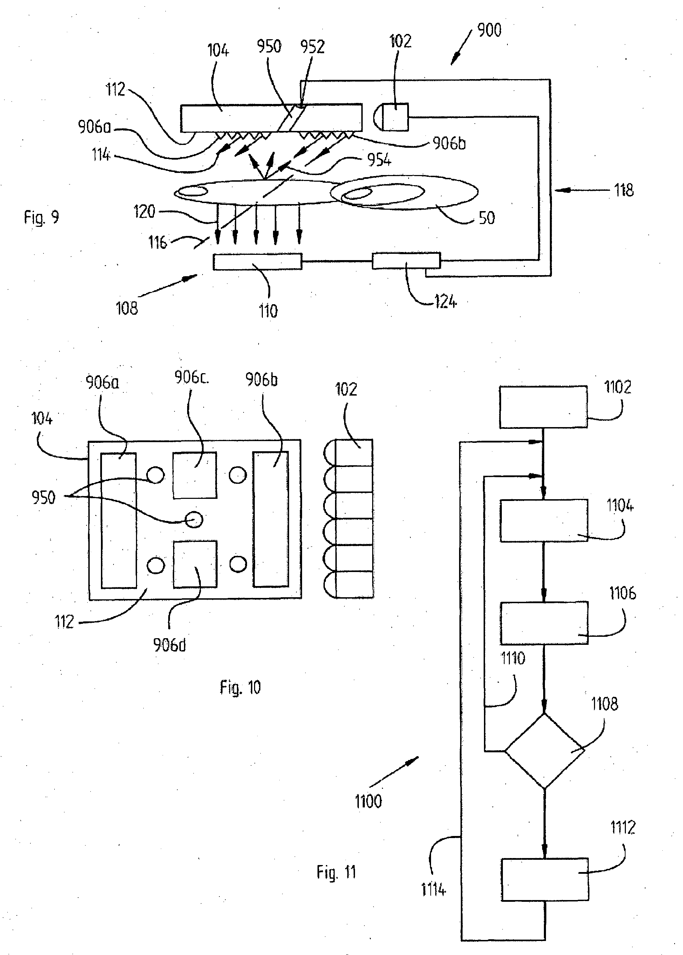

La Fig. 11 montre un algorithme d'un procédé d'asservissement 1100 selon l'invention qui peut être mis en oeuvre pour un capteur 700 représenté sur les Figs. 7 et 8.

Le procédé d'asservissement 1100 comprend:
- une étape d'initialisation 1102, au cours de laquelle le moyen de détection 750 envoie à l'unité de commande 124, la valeur de référence représentative de la puissance lumineuse qu'il reçoit en l'absence de la partie,
- une étape d'envoi 1104 au cours de laquelle le moyen de détection 750 envoie à l'unité de commande 124, une valeur représentative de la puissance lumineuse qu'il reçoit,
- une étape de calcul 1106 au cours de laquelle l'unité de commande 124 calcule l'écart entre la valeur ainsi reçue et la valeur de référence,
- une étape de test 1108, au cours de laquelle l'unité de commande 124 vérifie si l'écart ainsi calculé est supérieur, en valeur absolue, à un écart prédéterminé,
- si l'écart ainsi calculé reste inférieur à l'écart prédéterminé, une étape de bouclage 1110 au cours de laquelle le processus boucle sur l'étape d'envoi 1104,
- si l'écart ainsi calculé est supérieur à l'écart prédéterminé, une étape de commande 1112 au cours de laquelle l'unité de commande 124 commande une augmentation de la puissance lumineuse émise par la source d'éclairement 102, et
- une étape de retour 1114 au cours de laquelle le processus boucle sur l'étape d'envoi 1104.

La Fig. 9 et la Fig. 10 montrent un capteur 900 comportant également des moyens d'asservissement de la source d'éclairage 102 pour commander en puissance la source d'éclairement 102 selon que le doigt 50 soit ou non présent dans le passage 118.

Les moyens d'asservissement sont constitués ici par un ou plusieurs moyens de détection 952 et l'unité de commande 124.

Les moyens de détection 952 sont ici disposés au voisinage des zones d'extraction 906a-d.

En l'absence du doigt 50, les moyens de détection 952 ne reçoivent pratiquement aucun rayon infrarouge, tandis qu'en présence du doigt 50, les moyens de détection 952 captent alors les rayons infrarouges 954 rétrodiffusés par le doigt 50 vers le guide d'onde 104.

Ainsi, en fonction de la puissance lumineuse qu'ils reçoivent, les moyens de détection 952 envoient une valeur représentative de cette puissance lumineuse à l'unité de commande 124 qui, en fonction de cette valeur, commande la source d'éclairement 102 de manière à augmenter ou non la puissance lumineuse qu'elle émet grâce à des moyens appropriés qu'elle comporte.

Pour éviter que les moyens de détection 952 soient perturbés par les rayons infrarouges réfléchis par la peau du doigt 50, chacun est disposé au fond d'un trou 950 réalisé dans le guide d'onde 104, chacun desdits trous 950 présentant un axe sensiblement parallèle à la direction d'extraction 116 pour protéger les moyens de détection 952 des rayons infrarouges directement réfléchis par le doigt 50.

Pour éviter que les moyens de détection 952 soient perturbés par les rayons infrarouges traversant le guide d'onde 104, la surface desdits trous 950 est recouverte d'un matériau imperméable aux rayons infrarouges.

Une autre méthode d'asservissement peut consister en une analyse du niveau du moyen de contraste de l'image du réseau veineux capturé et de commander la puissance d'émission de la source d'éclairement 102 en fonction de ce niveau moyen.

Dans chaque mode de réalisation précédent, la face 112 où est disposée la zone d'extraction 106 constitue la face d'extraction 112 par où sont extraits les rayons infrarouges 114, 714a, 714b, et le moyen d'acquisition d'images 108 est disposé en vis-à-vis de la face d'extraction 112.

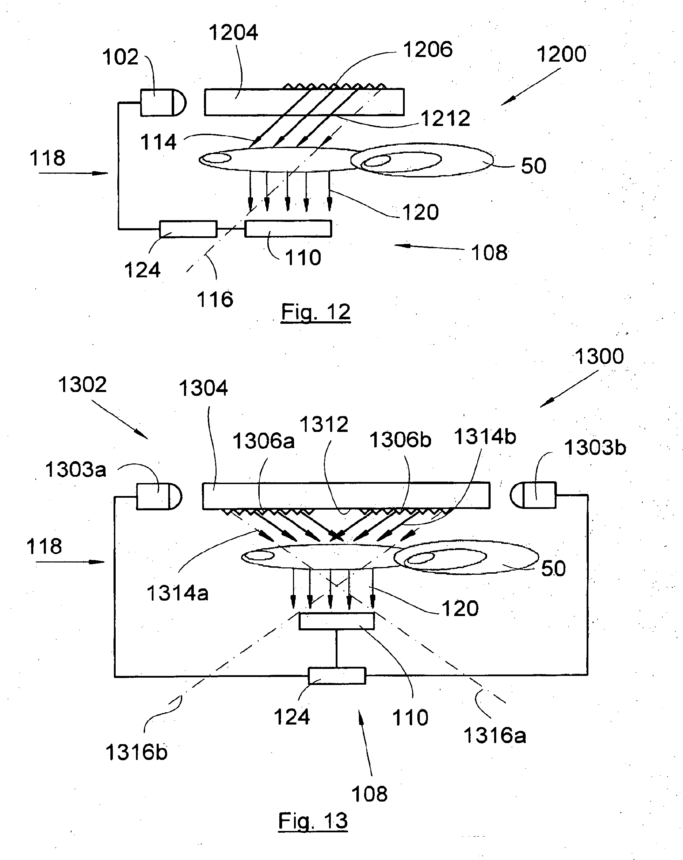

La Fig. 12 montre un capteur 1200 selon un neuvième mode de réalisation de l'invention qui comporte:
- une source d'éclairement infrarouge 102,
- un guide d'onde 1204 éclairé par la source d'éclairement infrarouge 102 et présentant au moins une zone d'extraction 1206 destinée à extraire les rayons infrarouges 114 du guide d'onde 1204 selon au moins une direction principale d'extraction 116 par une face d'extraction 1212 dudit guide d'onde 1204,
- un moyen d'acquisition d'images 108 disposé en vis-à-vis de ladite face d'extraction 1212 de manière à définir entre eux un passage 118 par l'entrée duquel ladite partie 50 du corps vivant peut pénétrer, et comportant un élément sensible 110,
la ou chaque zone d'extraction 1206 étant telle que la ou chaque direction principale d'extraction 116 est orientée de manière à ne pas intercepter ledit élément sensible 110.

Contrairement aux modes de réalisation précédents, la face d'extraction 1212 du capteur 1200 n'est pas la face qui porte la zone d'extraction 1206, mais la face opposée à la face qui porte la zone d'extraction 1206.

La zone d'extraction 1206 peut également se présenter sous la forme d'un patch qui est optiquement couplé à la face opposée à la face d'extraction 1212 du guide d'onde 1204 et il comporte ici des prismes, et plus particulièrement de microprismes qui sont utilisés en réflexion.

Dans ce mode de fonctionnement où la pente de chaque microprisme est de 70° par rapport à la normale de la face d'extraction 1212, la direction principale d'extraction 116 présente un angle d'environ 8° avec la normale de la face d'extraction 1212. Un tel positionnement de la zone d'extraction permet d'obtenir une nappe d'émission resserrée angulairement.

La surface en réflexion dans ce mode de réalisation est plus importante et les rendements de couplage photonique sont meilleurs avec des taux de fuite moindres.

La nappe générée subit globalement une réfraction supplémentaire qui augmente l'angle solide d'émission élémentaire.

Bien sûr, il est possible d'appliquer l'enseignement des modes de réalisation précédents au mode de réalisation de la Fig. 12. Par exemple, il est possible de disposer la source lumineuse 102 à l'opposé de l'entrée du passage 118 (Fig. 2) en disposant une surface réfléchissante présentant un angle d'inclinaison prévu pour replier les rayons infrarouges transmis et les dévier vers la ou chaque zone d'extraction 1206. Par exemple, il est possible de prévoir une mise en oeuvre similaire à celle de la Fig. 3. Il est également possible de mettre en place une plaque de protection placée en vis-à-vis de la face opposée à la face d'extraction 1212 et constituée d'un matériau empêchant le passage des rayons infrarouges mais laissant passer les rayons du spectre visible. Il est également possible de disposer une plaque de filtrage entre la zone d'extraction 1206 et le passage 118 et constituée d'un matériau empêchant le passage des rayons du spectre visible mais laissant passer les rayons infrarouges. Il est également possible de mettre en oeuvre des moyens d'asservissement prévus pour commander en puissance la source d'éclairement 102.

La Fig. 13 montre un capteur 1300 selon un dixième mode de réalisation de l'invention.

Le capteur 1300 comporte:
- une source d'éclairement infrarouge 1302, et
- un moyen d'acquisition d'images 108.

Le capteur 1300 comporte en outre un guide d'onde 1304 éclairé par ladite source d'éclairement infrarouge 1302 et présentant une première zone d'extraction 1306b et une deuxième zone d'extraction 1306a, chacune étant destinée à extraire les rayons infrarouges 1314a et 1314b du guide d'onde 1304 par une face d'extraction 1312 du guide d'onde 1304 selon une direction principale d'extraction respectivement référencée 1316a et 1316b.

Le moyen d'acquisition d'images 108 est disposé en vis-à-vis de la face d'extraction 1312 de manière à définir entre eux un passage 118 par l'entrée duquel la partie 50 du corps vivant peut pénétrer, et comporte un élément sensible 110.

Chaque zone d'extraction 1306a, 1306b est telle que chaque direction principale d'extraction 1316a, 1316b est orientée de manière à ne pas intercepter ledit élément sensible 110.

Dans le mode de réalisation de l'invention présenté sur la Fig. 13, chaque zone d'extraction 1306a, 1306b est portée par la face d'extraction 1312.

La source d'éclairement 1302 présente un premier élément éclairant 1303b éclairant la tranche du guide d'onde 1304 disposée du côté de l'entrée du passage 118, et un deuxième élément éclairant 1303a éclairant la tranche du guide d'onde 1304 disposée du côté opposé à l'entrée du passage 118. Les éléments éclairant 1303a et 1303b sont par exemple du type barrette de diodes.

Chaque zone d'extraction 1306a, 1306b est ici constituée de microprismes.

La première zone d'extraction 1306b est disposée entre le premier élément éclairant 1303b et la deuxième zone d'extraction 1306a, et elle est prévue pour extraire les rayons infrarouges 1314b issus du premier élément éclairant 1303b.

La deuxième zone d'extraction 1306a est disposée entre le deuxième élément éclairant 1303a et la première zone d'extraction 1306b, et elle est prévue pour extraire les rayons infrarouges 1314a issus du deuxième élément éclairant 1303a.

Les zones d'extraction 1306a et 1306b sont telles que les rayons infrarouges 1314b extraits du guide d'onde 1304 par la première zone d'extraction 1306b et les rayons infrarouges 1314a extraits du guide d'onde 1304 par la deuxième zone d'extraction 1306a convergent les uns vers les autres au niveau de l'endroit du passage 118 où est susceptible de se positionner la partie 50 du corps vivant et en amont du moyen d'acquisition d'images 108.

Il est ainsi possible d'équilibrer l'éclairage de la partie 50.

Bien entendu, la présente invention n'est pas limitée aux exemples et modes de réalisation décrits et représentés, mais elle est susceptible de nombreuses variantes accessibles à l'homme de l'art.

## Revendications

1. Capteur (100, 200, 300, 400, 500, 600, 700, 900, 1200, 1300) de réseaux veineux d'une partie (50) d'un corps vivant comportant:
- une source d'éclairement infrarouge (102, 1302), et
- un moyen d'acquisition d'images (108),
ledit capteur (100, 200, 300, 400, 500, 600, 700, 900, 1200, 1300) étant **caractérisé en ce qu'**il comporte en outre un guide d'onde (104, 204, 304, 404, 1204, 1304) éclairé par ladite source d'éclairement infrarouge (102, 1302) et présentant au moins une zone d'extraction (106, 450, 506, 906a-d, 1206, 1306a, 1306b) destinée à extraire les rayons infrarouges (114, 714a, 714b, 1314a, 1314b) dudit guide d'onde (104, 204, 304, 404, 1204, 1304) par une face d'extraction (112, 1212, 1312) du guide d'onde (104, 204, 304, 404, 1204, 1304) selon au moins une direction principale d'extraction (116, 452, 550, 1316, 1316b),
**en ce que** ledit moyen d'acquisition d'images (108) est disposé en vis-à-vis de ladite face d'extraction (112, 1212, 1312) de manière à définir entre eux un passage (118) par l'entrée duquel ladite partie (50) du corps vivant peut pénétrer, et comportant un élément sensible (110), et
**en ce que** la ou chaque zone d'extraction (106, 450, 506, 906a-d, 1206, 1306a, 1306b) est telle que la ou chaque direction principale d'extraction (116, 452, 550, 1316a, 1316b) est orientée de manière à ne pas intercepter ledit élément sensible (110).

2. Capteur (1200) selon la revendication 1, **caractérisé en ce que** ladite ou chaque zone d'extraction (1206) est portée par la face opposée à la face d'extraction (1212).

3. Capteur (100, 200, 300, 400, 500, 600, 700, 900, 1300) selon la revendication 1, **caractérisé en ce que** ladite ou chaque zone d'extraction (106, 506, 906a-d, 1306a, 1306b) est portée par la face d'extraction (112, 1312).

4. Capteur (100, 200, 300, 400, 500, 600, 700, 900) selon l'une des revendications 1 ou 3, **caractérisé en ce que** ladite ou au moins une desdites zones d'extraction (450) est constituée d'un pan incliné (450) réalisé dans ladite face d'extraction (112).

5. Capteur (100, 200, 300, 400, 500, 600, 700, 900, 1200, 1300) selon l'une des revendications 1 à 4, **caractérisé en ce que** ladite ou au moins une desdites zones d'extraction (106, 506, 906a-d, 1206, 1306a, 1306b) est constituée de microprismes.

6. Capteur (400) selon la revendication 5, lorsqu'elle dépend de la revendication 4, **caractérisé en ce que** le pan incliné (450) est en amont des microprismes dans le sens de progression des rayons infrarouges (122) dans le guide d'onde (404).

7. Capteur (200) selon l'une des revendications 1 à 6, **caractérisé en ce que** la source lumineuse (102) est disposée à l'opposé de l'entrée du passage (118), **en ce que** le capteur (200) comporte un moyen de collimation (252) disposé entre la source d'éclairement (102) et le guide d'onde (204) et prévu pour collimater au voisinage de l'infini, les rayons infrarouges issus de la source d'éclairement (102), et **en ce que** le guide d'onde (204) présente sur le trajet des rayons infrarouges (122) ainsi collimatés, une surface réfléchissante (250) présentant un angle d'inclinaison prévu pour replier les rayons infrarouges transmis (122) et les dévier vers la ou chaque zone d'extraction (106).

8. Capteur (300) selon l'une des revendications 1 à 6, **caractérisé en ce que** la source lumineuse (102) est disposée à l'opposé de l'entrée du passage (118), **en ce que** le capteur (300) comporte un moyen de focalisation (352) disposé entre la source d'éclairement (102) et le guide d'onde (304) et prévu pour focaliser les rayons infrarouges issus de la source d'éclairement (102) vers un point de focalisation (354), **en ce que** le guide d'onde (304) présente sur le trajet des rayons infrarouges (122) ainsi focalisés, une surface réfléchissante (350) disposée à distance du point de focalisation (354) et prévue pour réfléchir les rayons infrarouges (122) et provoquer leur déviation vers la zone d'extraction (106).

9. Capteur (1300) selon l'une des revendications 1 à 6, **caractérisé en ce que** ledit guide d'onde (1304) présente une première zone d'extraction (1306b) et une deuxième zone d'extraction (1306a), **en ce que** la ladite source d'éclairement infrarouge (1302) présente un premier élément éclairant (1303b) éclairant la tranche du guide d'onde (1304) disposée du côté de l'entrée du passage (118), et un deuxième élément éclairant (1303a) éclairant la tranche du guide d'onde (1304) disposée du côté opposé à l'entrée du passage (118), **en ce que** la première zone d'extraction (1306b) est disposée entre le premier élément éclairant (1303b) et la deuxième zone d'extraction (1306a) et est prévue pour extraire les rayons infrarouges (1314b) issus du premier élément éclairant (1303b), **en ce que** la deuxième zone d'extraction (1306a) est disposée entre le deuxième élément éclairant (1303a) et la première zone d'extraction (1306b), et est prévue pour extraire les rayons infrarouges (1314a) issus du deuxième élément éclairant (1303a), et **en ce que** les zones d'extraction (1306a, 1306b) sont telles que les rayons infrarouges (1314b) extraits par la première zone d'extraction (1306b) et les rayons infrarouges (1314a) extraits par la deuxième zone d'extraction (1306a) convergent les uns vers les autres au niveau de l'endroit du passage (118) où est susceptible de se positionner la partie (50).

10. Capteur (600) selon l'une des revendications précédentes, **caractérisé en ce qu'**il comporte une plaque de protection (650) placée en vis-à-vis de la face opposée à la face d'extraction (112) et constituée d'un matériau empêchant le passage des rayons infrarouges mais laissant passer les rayons du spectre visible.

11. Capteur (600) selon l'une des revendications précédentes, **caractérisé en ce qu'**il comporte une plaque de filtrage (652) placée entre la zone d'extraction (106) et le passage (118) et constituée d'un matériau empêchant le passage des rayons du spectre visible mais laissant passer les rayons infrarouges.

12. Capteur (700, 900) selon l'une des revendications précédentes, **caractérisé en ce qu'**il comporte des moyens d'asservissement (750, 952, 124) prévus pour commander en puissance la source d'éclairement (102).

13. Capteur (700, 900) selon la revendication 12, **caractérisé en ce que** les moyens d'asservissement sont constitués par une unité de commande (124) prévue pour commander la source d'éclairement (102) et au moins un moyen de détection (750, 952) prévu pour envoyer à ladite unité de commande (124), une valeur représentative de la puissance d'éclairement qu'il reçoit, l'unité de commande (124) comportant des moyens pour commander la source d'éclairement (102) en fonction de ladite valeur.

14. Capteur (700) selon l'une des revendications 12 ou 13, **caractérisé en ce que** le ou chaque moyen de détection (750) est placé au voisinage de l'élément sensible (110).

15. Capteur (900) selon l'une des revendications 12 ou 13, **caractérisé en ce que** le ou chaque moyen de détection (952) est disposé au voisinage de la ou des zones d'extraction (906a-d).

16. Capteur (900) selon la revendication 15, **caractérisé en ce que** le ou chaque moyen de détection (952) est disposé au fond d'un trou réalisé dans le guide d'onde (104), chacun desdits trous (950) présentant un axe sensiblement parallèle à la direction d'extraction (116).

17. Capteur (900) selon la revendication 16, **caractérisé en ce que** la surface du ou de chaque trou (950) est recouverte d'un matériau imperméable aux rayons infrarouges.

18. Procédé d'asservissement (1100) d'un capteur (700) selon la revendication 13, ledit procédé (1100) comprenant:
- une étape d'initialisation (1102), au cours de laquelle le moyen de détection (750) envoie à l'unité de commande (124), la valeur de référence représentative de la puissance lumineuse qu'il reçoit en l'absence de la partie,
- une étape d'envoi (1104) au cours de laquelle le moyen de détection (750) envoie à l'unité de commande (124), une valeur représentative de la puissance lumineuse qu'il reçoit,
- une étape de calcul (1106) au cours de laquelle l'unité de commande (124) calcule l'écart entre la valeur ainsi reçue et la valeur de référence,
- une étape de test (1108), au cours de laquelle l'unité de commande (124) vérifie si l'écart ainsi calculé est supérieur, en valeur absolue, à un écart prédéterminé,
- si l'écart ainsi calculé reste inférieur à l'écart prédéterminé, une étape de bouclage (1110) au cours de laquelle le processus boucle sur l'étape d'envoi (1104),
- si l'écart ainsi calculé est supérieur à l'écart prédéterminé, une étape de commande (1112) au cours de laquelle l'unité de commande (124) commande une augmentation de la puissance lumineuse émise par la source d'éclairement (102), et
- une étape de retour (1114) au cours de laquelle le processus boucle sur l'étape d'envoi (1104).

## Patentansprüche

1. Sensor (100, 200, 300, 400, 500, 600, 700, 900, 1200, 1300) für Venennetze eines Teils (50) eines lebenden Körpers, wobei der Sensor Folgendes umfasst:
- eine Infrarotbeleuchtungsquelle (102, 1302) und
- ein Bilderfassungsmittel (108),
wobei der Sensor (100, 200, 300, 400, 500, 600, 700, 900, 1200, 1300) **dadurch gekennzeichnet** ist, dass er außerdem einen Wellenleiter (104, 204, 304, 404, 1204, 1304) umfasst, der von der Infrarotbeleuchtungsquelle (102, 1302) beleuchtet wird und mindestens eine Extraktionszone (106, 450, 506, 906a-d, 1206, 1306a, 1306b) aufweist, die zum Extrahieren der Infrarotstrahlen (114, 714a, 714b, 1314a, 1314b) aus dem Wellenleiter (104, 204, 304, 404, 1204, 1304) durch eine Extraktionsfläche (112, 1212, 1312) des Wellenleiters (104, 204, 304, 404, 1204, 1304) gemäß mindestens einer Hauptextraktionsrichtung (116, 452, 550, 1316, 1316b) vorgesehen ist,
dass das Bilderfassungsmittel (108) derart gegenüber der Extraktionsfläche (112, 1212, 1312) angeordnet ist, dass zwischen ihnen ein Durchgang (118) definiert wird, durch dessen Eintritt der Teil (50) des lebenden Körpers eindringen kann, und ein empfindliches Element (110) umfasst, und
dass die oder jede Extraktionszone (106, 450, 506, 906a-d, 1206, 1306a, 1306b) derart ist, dass die oder jede Hauptextraktionsrichtung (116, 452, 550, 1316a, 1316b) derart ausgerichtet ist, dass das empfindliche Element (110) nicht unterbrochen wird.

2. Sensor (1200) nach Anspruch 1, **dadurch gekennzeichnet, dass** die oder jede Extraktionszone (1206) von der Fläche getragen wird, die der Extraktionsfläche (1212) gegenüberliegt.

3. Sensor (100, 200, 300, 400, 500, 600, 700, 900, 1300) nach Anspruch 1, **dadurch gekennzeichnet, dass** die oder jede Extraktionszone (106, 506, 906a-d, 1306a, 1306b) von der Extraktionsfläche (112, 1312) getragen wird.

4. Sensor (100, 200, 300, 400, 500, 600, 700, 900) nach Anspruch 1 oder 3, **dadurch gekennzeichnet, dass** die oder mindestens eine der Extraktionszonen (450) aus einer geneigten Kante (450) besteht, die in der Extraktionsfläche (112) ausgebildet ist.

5. Sensor (100, 200, 300, 400, 500, 600, 700, 900, 1200, 1300) nach einem der Ansprüche 1 bis 4, **dadurch gekennzeichnet, dass** die oder mindestens eine der Extraktionszonen (106, 506, 906a-d, 1206, 1306a, 1306b) aus Mikroprismen besteht.

6. Sensor (400) nach Anspruch 5 bei Abhängigkeit von Anspruch 4, **dadurch gekennzeichnet, dass** die geneigte Kante (450) vor Mikroprismen in der Verlaufsrichtung von Infrarotstrahlen (122) in dem Wellenleiter (404) liegt.

7. Sensor (200) nach einem der Ansprüche 1 bis 6, **dadurch gekennzeichnet, dass** die Leuchtquelle (102) gegenüber dem Eintritt des Durchgangs (118) angeordnet ist, dass der Sensor (200) ein Bündelungsmittel (252) umfasst, das zwischen der Beleuchtungsquelle (102) und dem Wellenleiter (204) angeordnet ist und dazu vorgesehen ist, die von der Beleuchtungsquelle (102) abgegebenen Infrarotstrahlen gegen unendlich zu bündeln, und dass der Wellenleiter (204) auf dem Weg der so gebündelten Infrarotstrahlen (122) eine reflektierende Oberfläche (250) aufweist, die einen Neigungswinkel aufweist, der dazu vorgesehen ist, die übertragenen Infrarotstrahlen (122) zu beugen und sie zu der oder jeder Extraktionszone (106) umzuleiten.

8. Sensor (300) nach einem der Ansprüche 1 bis 6, **dadurch gekennzeichnet, dass** die Leuchtquelle (102) gegenüber dem Eintritt des Durchgangs (118) angeordnet ist, dass der Sensor (300) ein Fokussierungsmittel (352) umfasst, das zwischen der Beleuchtungsquelle (102) und dem Wellenleiter (304) angeordnet ist und dazu vorgesehen ist, die von der Beleuchtungsquelle (102) abgegebenen Infrarotstrahlen zu einem Fokussierpunkt (354) zu fokussieren, und dass der Wellenleiter (304) auf dem Weg der so fokussierten Infrarotstrahlen (122) eine reflektierende Oberfläche (350) aufweist, die in einem Abstand von dem Fokussierpunkt (354) angeordnet ist und dazu vorgesehen ist, die Infrarotstrahlen (122) zu reflektieren und ihre Umleitung zu der Extraktionszone (106) zu bewirken.

9. Sensor (1300) nach einem der Ansprüche 1 bis 6, **dadurch gekennzeichnet, dass** der Wellenleiter (1304) eine erste Extraktionszone (1306b) und eine zweite Extraktionszone (1306a) aufweist, dass die Infrarotbeleuchtungsquelle (1302) ein erstes Leuchtelement (1303b) aufweist, das den Rand des Wellenleiters (1304) beleuchtet und an der Seite des Eintritts des Durchgangs (118) angeordnet ist, und ein zweites Leuchtelement (1303a), das den Rand des Wellenleiters (1304) beleuchtet und an der Seite angeordnet ist, die dem Eintritt des Durchgangs (118) gegenüberliegt, dass die erste Extraktionszone (1306b) zwischen dem ersten Leuchtelement (1303b) und der zweiten Extraktionszone (1306a) angeordnet ist und dazu vorgesehen ist, die von dem ersten Leuchtelement (1303b) abgegebenen Infrarotstrahlen (1314b) zu extrahieren, dass die zweite Extraktionszone (1306a) zwischen dem zweiten Leuchtelement (1303a) und der ersten Extraktionszone (1306b) angeordnet ist und dazu vorgesehen ist, die von dem zweiten Leuchtelement (1303a) abgegebenen Infrarotstrahlen (1314a) zu extrahieren, und dass die Extraktionszonen (1306a, 1306b) derart sind, dass die von der ersten Extraktionszone (1306b) extrahierten Infrarotstrahlen (1314b) und die von der zweiten Extraktionszone (1306a) extrahierten Infrarotstrahlen (1314a) auf Höhe der Stelle des Durchgangs (118), die für das Positionieren des Teils (50) empfänglich ist, zueinander konvergieren.

10. Sensor (600) nach einem der vorhergehenden Ansprüche, **dadurch gekennzeichnet, dass** er eine Schutzplatte (650) umfasst, die gegenüber der Gegenseite der Extraktionsfläche (112) positioniert ist und aus einem Material besteht, das den Durchtritt von Infrarotstrahlen verhindert, jedoch die Strahlen des sichtbaren Spektrums durchtreten lässt.

11. Sensor (600) nach einem der vorhergehenden Ansprüche, **dadurch gekennzeichnet, dass** er eine Filterplatte (652) umfasst, die zwischen der Extraktionszone (106) und dem Durchgang (118) positioniert ist und aus einem Material besteht, das den Durchtritt von Strahlen des sichtbaren Spektrums verhindert, jedoch die Infrarotstrahlen durchtreten lässt.

12. Sensor (700, 900) nach einem der vorhergehenden Ansprüche, **dadurch gekennzeichnet, dass** er Regelungsmittel (750, 952, 124) umfasst, die dazu vorgesehen sind, die Leistung der Beleuchtungsquelle (102) zu steuern.

13. Sensor (700, 900) nach Anspruch 12, **dadurch gekennzeichnet, dass** die Regelungsmittel aus einer Steuereinheit (124) bestehen, die dazu vorgesehen ist, die Beleuchtungsquelle (102) zu steuern, und mindestens einem Detektionsmittel (750, 952), das dazu vorgesehen ist, der Steuereinheit (125) einen Wert zu senden, der die Beleuchtungsleistung darstellt, die es empfängt, wobei die Steuereinheit (124) Mittel zum Steuern der Beleuchtungsquelle (102) in Abhängigkeit von dem Wert umfasst.

14. Sensor (700) nach einem der Ansprüche 12 oder 13, **dadurch gekennzeichnet, dass** das oder jedes Detektionsmittel (750) in der Nähe des empfindlichen Elements (110) positioniert ist.

15. Sensor (900) nach einem der Ansprüche 12 oder 13, **dadurch gekennzeichnet, dass** das oder jedes Detektionsmittel (952) in der Nähe der Extraktionszone bzw. der Extraktionszonen (906a-d) positioniert ist.

16. Sensor (900) nach Anspruch 15, **dadurch gekennzeichnet, dass** das oder jedes Detektionsmittel (952) auf dem Boden eines Lochs angeordnet ist, das in dem Wellenleiter (104) ausgebildet ist, wobei jedes der Löcher (950) eine Achse aufweist, die zu der Extraktionsrichtung (116) im Wesentlichen parallel ist.

17. Sensor (900) nach Anspruch 16, **dadurch gekennzeichnet, dass** die Oberfläche des oder jedes Lochs (950) mit einem Material bedeckt ist, das für die Infrarotstrahlen undurchlässig ist.

18. Verfahren zur Regelung (1100) eines Sensors (700) nach Anspruch 13, wobei das Verfahren (1100) folgendes umfasst:
- einen Initialisierungsschritt (1102), in dem das Detektionsmittel (750) der Steuereinheit (124) den Bezugswert sendet, der die Leuchtleistung darstellt, die es in Abwesenheit des Teils empfängt,
- einen Sendeschritt (1104), in dem das Detektionsmittel (750) der Steuereinheit (124) einen Wert sendet, der die Leuchtleistung darstellt, die es empfängt,
- einen Berechnungsschritt (1106), in dem die Steuereinheit (124) den Unterschied zwischen dem so empfangenen Wert und dem Bezugswert berechnet,
- einen Testschritt (1108), in dem die Steuereinheit (124) prüft, ob der so berechnete Unterschied als absoluter Wert höher als ein vorher bestimmter Unterschied ist,
- wenn der so berechnete Unterschied unter dem vorher bestimmten Unterschied bleibt, einen Schleifendurchlaufschritt (1110), in dem der Vorgang eine Schleife zurück zu dem Sendeschritt (1104) vornimmt,
- wenn der so berechnete Unterschied höher als der vorher bestimmte Unterschied ist, einen Steuerungsschritt (1112), in dem die Steuereinheit (124) eine Erhöhung der Leuchtleistung steuert, die von der Beleuchtungsquelle (102) abgestrahlt wird, und
- einen Rückkehrschritt (1114), in dem der Vorgang eine Schleife zurück zu dem Sendeschritt (1104) vornimmt.

## Claims

1. Sensor (100, 200, 300, 400, 500, 600, 700, 900, 1200, 1300) for venous networks of a part (50) of a living body, comprising:
- an infra-red illumination source (102, 1302) and
- an image acquisition means (108),
said sensor (100, 200, 300, 400, 500, 600, 700, 900, 1200, 1300) being **characterised in that** it further comprises a waveguide (104, 204, 304, 404, 1204, 1304) which is illuminated by said infra-red illumination source (102, 1302) and has at least one extraction zone (106, 450, 506, 906a-d, 1206, 1306a, 1306b) which is to extract the infra-red rays (114, 714a, 714b, 1314a, 1314b) from said waveguide (104, 204, 304, 404, 1204, 1304) through an extraction face (112, 1212, 1312) of the waveguide (104, 204, 304, 404, 1204, 1304) in at least one main extraction direction (116, 452, 550, 1316, 1316b),
**in that** said image acquisition means (108) is arranged opposite said extraction face (112, 1212, 1312) so as to define between them a passage (118) through the entrance of which said part (50) of the living body is able to enter, and comprising a sensitive element (110), and
**in that** the or each extraction zone (106, 450, 506, 906a-d, 1206, 1306a, 1306b) is such that the or each main extraction direction (116, 452, 550, 1316a, 1316b) is oriented so that it does not intercept said sensitive element (110).

2. Sensor (1200) according to claim 1, **characterised in that** said or each extraction zone (1206) is carried by the face opposite the extraction face (1212).

3. Sensor (100, 200, 300, 400, 500, 600, 700, 900, 1300) according to claim 1, **characterised in that** said or each extraction zone (106, 506, 906a-d, 1306a, 1306b) is carried by the extraction face (112, 1312).

4. Sensor (100, 200, 300, 400, 500, 600, 700, 900) according to either claim 1 or claim 3, **characterised in that** said extraction zone or at least one of said extraction zones (450) is constituted by an inclined portion (450) formed in said extraction face (112).

5. Sensor (100, 200, 300, 400, 500, 600, 700, 900, 1200, 1300) according to any one of claims 1 to 4, **characterised in that** said extraction zone or at least one of said extraction zones (106, 506, 906a-d, 1206, 1306a, 1306b) is constituted by microprisms.

6. Sensor (400) according to claim 5, when it is dependent on claim 4, **characterised in that** the inclined portion (450) is upstream of the microprisms in the direction of progression of the infra-red rays (122) in the waveguide (404).

7. Sensor (200) according to any one of claims 1 to 6, **characterised in that** the light source (102) is arranged opposite the entrance of the passage (118), **in that** the sensor (200) comprises a collimation means (252) which is arranged between the illumination source (102) and the waveguide (204) and is provided to collimate in the vicinity of infinity the infra-red rays coming from the illumination source (102), and **in that** the waveguide (204) has in the path of the infra-red rays (122) so collimated a reflecting surface (250) having an angle of inclination which is provided to bend the transmitted infra-red rays (122) and deflect them towards the or each extraction zone (106).

8. Sensor (300) according to any one of claims 1 to 6, **characterised in that** the illumination source (102) is arranged opposite the entrance of the passage (118), **in that** the sensor (300) comprises a focusing means (352) which is arranged between the illumination source (102) and the waveguide (304) and is provided to focus the infra-red rays coming from the illumination source (102) towards a focusing point (354), **in that** the waveguide (304) has in the path of the infra-red rays (122) so focused a reflecting surface (350) which is arranged at a distance from the focusing point (354) and is provided to reflect the infra-red rays (122) and cause the deflection thereof towards the extraction zone (106).

9. Sensor (1300) according to any one of claims 1 to 6, **characterised in that** said waveguide (1304) has a first extraction zone (1306b) and a second extraction zone (1306a), **in that** said infra-red illumination source (1302) has a first illuminating element (1303b) which illuminates the edge of the waveguide (1304) that is arranged on the side of the entrance of the passage (118) and a second illuminating element (1303a) which illuminates the edge of the waveguide (1304) that is arranged on the side opposite the entrance of the passage (118), **in that** the first extraction zone (1306b) is arranged between the first illuminating element (1303b) and the second extraction zone (1306a) and is provided to extract the infra-red rays (1314b) coming from the first illuminating element (1303b), **in that** the second extraction zone (1306a) is arranged between the second illuminating element (1303a) and the first extraction zone (1306b) and is provided to extract the infra-red rays (1314a) coming from the second illuminating element (1303a), and **in that** the extraction zones (1306a, 1306b) are such that the infra-red rays (1314b) extracted by the first extraction zone (1306b) and the infra-red rays (1314a) extracted by the second extraction zone (1306a) converge towards one another at the point of the passage (118) where the part (50) is likely to be positioned.

10. Sensor (600) according to any one of the preceding claims, **characterised in that** it comprises a protective plate (650) which is located opposite the face opposite the extraction face (112) and is composed of a material which prevents the infra-red rays from passing but allows the rays of the visible spectrum to pass.

11. Sensor (600) according to any one of the preceding claims, **characterised in that** it comprises a filtering plate (652) which is located between the extraction zone (106) and the passage (118) and is composed of a material which prevents the rays of the visible spectrum from passing but allows the infra-red rays to pass.

12. Sensor (700, 900) according to any one of the preceding claims, **characterised in that** it comprises control means (750, 952, 124) which are provided to command the illumination source (102) in terms of power.

13. Sensor (700, 900) according to claim 12, **characterised in that** the control means are composed of a command unit (124), which is provided to command the illumination source (102), and at least one detection means (750, 952), which is provided to send to said command unit (124) a value representing the luminous power that it receives, the command unit (124) comprising means for commanding the illumination source (102) as a function of said value.

14. Sensor (700) according to either claim 12 or claim 13, **characterised in that** the or each detection means (750) is located in the vicinity of the sensitive element (110).

15. Sensor (900) according to either claim 12 or claim 13, **characterised in that** the or each detection means (952) is arranged in the vicinity of the extraction zone or zones (906a-d).

16. Sensor (900) according to claim 15, **characterised in that** the or each detection means (952) is arranged at the bottom of a hole formed in the waveguide (104), each of said holes (950) having an axis substantially parallel to the extraction direction (116).

17. Sensor (900) according to claim 16, **characterised in that** the surface of the or of each hole (950) is covered with a material that is impermeable to infra-red rays.

18. Method for controlling (1100) a sensor (700) according to claim 13, said method (1100) comprising:
- an initialisation step (1102), during which the detection means (750) sends to the command unit (124) the reference value representing the luminous power that it receives in the absence of the part,
- a sending step (1104), during which the detection means (750) sends to the command unit (124) a value representing the luminous power that it receives,
- a calcufation step (1106), during which the command unit (124) calculates the difference between the value so received and the reference value,
- a test step (1108), during which the command unit (124) checks whether the difference so calculated is greater, in absolute terms, than a predetermined difference,
- if the difference so calculated remains below the predetermined difference, a looping step (1110), during which the process loops back to the sending step (1104),
- if the difference so calculated is greater than the predetermined difference, a command step (1112), during which the command unit (124) commands an increase in the luminous power emitted by the illumination source (102), and
- a return step (1114), during which the process loops back to the sending step (1104).
